# EUROPÄISCHE PATENTANMELDUNG

(11) **EP 3 988 134 A1**
(43) Veröffentlichungstag der Anmeldung: **27.04.2022**
(21) Anmeldenummer: 20202880.9
(22) Anmeldetag: 20.10.2020
(51) Int. Cl.: A61L 9/12, A61L 2/22, F24F 6/12, F24F 8/20, F24F 8/24

(54) **VERFAHREN ZUR SANITATION ODER DESINFEKTION MIT WENIGSTENS EINEM AEROSOL, DAS TRÖPFCHEN EINES DESINFEKTIONSMITTELS ENTHÄLT**

(71) Anmelder: Boga GmbH Gesellschaft für Moderne Gerätetechnik, 59494 Soest (DE)
(72) Erfinder: DAHLHOFF, Joscha, 59510 Lippetal Brockhausen (DE)
(74) Vertreter: Schäperklaus, Jochen

(57) **Zusammenfassung**

Die Erfindung betrifft ein Verfahren zur Sanitation oder Desinfektion in einem Raum (1) mit wenigstens einem Aerosol, das Tröpfchen eines flüssigen Desinfektionsmittels enthält, wobei
- eine Konzentration (y(t)) des Desinfektionsmittels in der Luft des Raumes (1) unmittelbar oder mittelbar geregelt wird,
- eine zu erreichende Konzentration des Desinfektionsmittels in der Luft des Raumes (1) als Führungsgröße (w(t)) festgelegt wird,
- eine Konzentration (y(t)) des Desinfektionsmittels in der Luft des Raumes (1) mit wenigstens einem Sensor (2) unmittelbar oder mittelbar kontinuierlich oder zu diskreten Zeitpunkten erfasst wird und eine Regelgröße bildet,
- aus der erfassten Konzentration (y(t)) und der zu erreichenden Konzentration (w(t)) mit einem Regelungsmittel (3) kontinuierlich oder zu diskreten Zeitpunkten wenigstens eine kontinuierliche oder diskrete Stellgröße (u(t)) ermittelt wird,
- an wenigstens einem Mittel (4) zur Erzeugung des Aerosols fortlaufend die kontinuierliche oder diskrete Stellgröße (u(t)) eingestellt wird, das wenigstens eine Mittel (4) zur Erzeugung des Aerosols dann entsprechend der Einstellung aus einer einem Vorrat des Desinfektions- oder Sanitationsmittels entnommenen Menge des Desinfektions- oder Sanitationsmittels und aus dem Raum zugeführter Luft das Aerosol erzeugt und das erzeugte Aerosol in den Raum (1) austrägt,
- wobei die zu erreichende Konzentration (w(t)) des Desinfektionsmittels in der Luft des Raumes (1) geringer oder genauso groß ist wie ein Grenzwert für die Aerosolkonzentration für das Desinfektionsmittel. Mit der Erfindung soll die Desinfektion in Räumen während einer laufenden Nutzung der Räume ermöglicht werden.

## Beschreibung

Die vorliegende Erfindung betrifft ein Verfahren zur Sanitation oder Desinfektion in Räumen mit wenigstens einem Aerosol, das Tröpfchen eines Desinfektionsmittels enthält. Die Erfindung betrifft auch eine Vorrichtung zur Durchführung des Verfahrens.

Desinfektion bedeutet totes oder lebendes Material in einen Zustand zu versetzen, dass es nicht mehr infizieren kann. Die Sanitation ist dagegen der Einsatz von Methoden, die über die normalen Reinigungsmaßnahmen hinausgehen und zur möglichst weitgehenden Verminderung evtl. krank machender Keime und anderer Mikroorganismen führen, ohne dass das Ergebnis der Desinfektion erreicht werden muss.

In dem Dokument WO 2013/043409 A1 ist beschrieben, dass hohe Konzentrationen eines Desinfektionsmittels verwendet werden, um die Oberflächen in einem Raum zu desinfizieren. Während der Desinfektion kann der Raum durch Menschen nicht genutzt werden, da die Gefahr besteht, dass das Desinfektionsmittel den oder die Menschen schädigt. Nach der Desinfektion wird das Desinfektionsmittel aus der Raumluft entfernt. Erst wenn die Konzentration des Desinfektionsmittels so weit abgesunken ist, dass keine Gefahr durch das Desinfektionsmittel besteht, darf der Raum wieder von Menschen betreten werden. Eine Desinfektion bei laufender Nutzung des zu desinfizierenden Raums ist nicht erlaubt.

Eine Desinfektion mit einem Aerosol, das Tröpfchen eines Desinfektionsmittels enthält, ist effektiv, da Aerosole bei hinreichender Einwirkzeit alle, auch schwer zu erreichenden Oberflächen erreichen und sich Tröpfchen aus den Aerosolen auf den auch schwer zu erreichenden Oberflächen niederschlagen und damit Keime auf diesen Oberflächen abtöten können. Wasserstoffperoxidlösungen sind Desinfektionsmittel, aus denen häufig Aerosole erzeugt werden, die zur Desinfektion von Oberflächen in Räumen eingesetzt werden.

Um mit den bekannten Verfahren Räume mit Aerosolen zu desinfizieren ist man darauf angewiesen, die Nutzung des Raumes zu sonst üblichen Nutzungszeiten zu unterlassen oder die Desinfektion in Zeiten durchzuführen, in denen keine Nutzung des Raumes erfolgt. Da manche Räume ohne Unterbrechung genutzt werden und genutzt werden müssen, ist in diesen Räumen eine Desinfektion mit Aerosolen nicht möglich. Dann ist man auf andere Arten der Oberflächendesinfektion angewiesen die aufwändiger sind, zum Beispiel wischen. Aber selbst, wenn es Zeiten gibt, in denen der Raum nicht genutzt wird, ist man für die Desinfektion auf eben diese Zeiten beschränkt. Eine Desinfektion ist dann immer nur dann ohne Einschränkung der Nutzungszeit möglich, wenn keine Nutzungszeit ist, aber nicht, wenn es aus hygienischen Gründen sinnvoll ist. Auch in solchen Fällen wäre man dann auf eine Oberflächendesinfektion durch Wischen angewiesen.

Hier setzt die vorliegende Erfindung an.

Mit der Erfindung soll die Sanitation oder Desinfektion in Räumen mit wenigstens einem Aerosol, das Tröpfchen eines Desinfektionsmittels enthält, während einer laufenden Nutzung der Räume ermöglicht werden.

Eine Lösung dieses Problems bietet das erfindungsgemäße Verfahren mit den Merkmalen des Anspruchs 1. Im Unterschied zu den bekannten Verfahren zur Desinfektion mit Aerosolen, bei denen eine Desinfektion nur möglich ist, wenn der Raum nicht genutzt wird, kann das erfindungsgemäße Verfahren auch während einer laufenden Nutzung des Raums angewendet werden. Das ist möglich, weil die Grenzwerte, ab denen eine Konzentration des Desinfektionsmittels in der Raumluft als gesundheitsgefährdend angesehen wird, nicht überschritten wird. Dieser Grenzwert für die Konzentration des Desinfektionsmittels kann zum Beispiel aus folgenden Grenzwerten gewählt werden, die von verschiedenen Institutionen festgelegt und veröffentlicht werden: Maximale Arbeitsplatz-Konzentration (MAK-Wert), Arbeitsplatzgrenzwert (AGW), DNEL (derived no-effect level), DMEL (Derived Minimum Effect Level), Occupational Exposure Limits (OELs), indicative occupational exposure limit values (IOELVs), permissible exposure limit (PEL or OSHA PEL), recommended exposure limit (REL), threshold limit value (TLV). Es können auch andere Grenzwerte verwendet werden. Bevorzugt werden solche Grenzwerte verwendet, die von allgemein anerkannten Organisationen oder Behörden festgelegt und veröffentlicht werden.

Im Vergleich zu einer Desinfektion mit höheren Konzentrationen von Desinfektionsmittel, wie sie aus dem Stand der Technik bekannt ist, dauert die Desinfektion nach dem erfindungsgemäßen Verfahren länger, zum Teil deutlich länger. Da aber das Verfahren ohne Unterbrechung durch eine Nutzung des Raums ständig durchgeführt werden kann, hat sie aber gegenüber einer Desinfektion, die ausschließlich außerhalb der Nutzungszeiten eines Raumes durchgeführt werden kann, Vorteile.

So werden Keime, die sich während der Nutzung auf Oberflächen ansiedeln ohne zeitliche Verzögerung durch die andauernde Nutzung schon während der andauernden Nutzung mit geringen Dosen des Desinfektionsmittels bekämpft werden. Diese Bekämpfung kann, wenn sie aufgrund der geringen Wirkstoffkonzentration in der Raumluft und im Niederschlag auf die zu desinfizierende Fläche auch nicht zu einer schnellen Abtötung der Keime führt, zu einer schnellen Hemmung der Vermehrung der Keime und/oder einer Hemmung der Entwicklung der Keime und/oder einer Hemmung der Infektiosität oder Gefährlichkeit der Keime führen, was einen Zeitgewinn gegenüber der Vorgehensweise ist, bei der die Keime sich auf der Oberfläche zunächst ungehemmt vermehren können, bevor dann nach der Nutzung mit der Bekämpfung der Keime begonnen wird.

Außerdem ist es möglich, dass die zwischen der Kontaminierung der Oberflächen mit Keimen und dem Ende der Nutzungszeit liegende Zeit ausreicht, um die Keime mit der geringen Wirkstoffkonzentration abzutöten. Dann wäre eine Desinfektion schon vor der Zeit erreicht, zu der eine Desinfektion mit hohen Konzentrationen des Desinfektionsmittels begonnen werden könnte.

Der Zeitgewinn, der durch die sofort nach der Kontamination einsetzende Bekämpfung der Keime entsteht, kann dazu führen, dass die Infektion von Personen, die die kontaminierte Oberfläche berühren, verhindert wird oder aber die Infektion der Person mit nur einer geringen Keimzahl erfolgt, so dass die durch die Keime ausgelöste Krankheit einen milderen Verlauf hat, als es bei einer Infizierung mit einer großen Anzahl von Keimen der Fall wäre.

Die Anwesenheit von Aerosoltröpfchen, die ein Desinfektionsmittel enthalten, in der Luft eines Raumes während der Nutzung des Raumes kann aber auch aus einem weiteren Grund einen Vorteil haben. Kommt es in dem Raum zu einer Verbreitung von mit Keimen kontaminierten Aerosoltröpfchen, zum Beispiel durch eine erkrankte Person, durch einen Wasserauslauf (zum Beispiel eine Brause, einen Springbrunnen), einen Luftbefeuchter oder ähnliches, schlagen sich die kontaminierten Aerosoltröpfchen auf den Oberflächen des Raumes nieder und kontaminieren diese. Zusätzlich besteht aber auch die Gefahr, dass die kontaminierten Aerosoltröpfchen von Personen in dem Raum eingeatmet werden und diese einatmenden Personen infizieren. Sind aber gleichzeitig Aerosoltröpfchen in der Luft des Raumes, die ein Desinfektionsmittel enthalten, können die kontaminierten Tröpfchen und die das Desinfektionsmittel enthaltenden Tröpfchen aufeinandertreffen und sich zu einem größeren Tröpfchen vereinen. Die Keime treffen dann in den größeren Tröpfchen auf das Desinfektionsmittel wodurch es dazu kommen kann, dass die Keime durch das Desinfektionsmittel getötet werden, bevor sie eingeatmet werden oder sich auf einer Oberfläche niederschlagen. Dadurch wird eine Kontaminierung der Oberflächen oder eine Infektion durch Einatmen schon im Ansatz verhindert.

Die Konzentration des Desinfektionsmittels in der Luft kann bei einem erfindungsgemäßen Verfahren unmittelbar und mittelbar gemessen werden. Unmittelbar bedeutet, dass der Sensor oder die Sensoren zum Erfassen der Konzentration des Desinfektionsmittels in dem Raum angeordnet sind. Mittelbar bedeutet, dass der Sensor oder die Sensoren zum Erfassen der Konzentration des Desinfektionsmittels in einem Kanal in dem Mittel zur Erzeugung des Aerosols angeordnet ist, vorzugsweise in einem Kanal durch den das erzeugte Aerosol zu einem Auslass geführt wird, durch den das Aerosol aus dem Mittel zur Erzeugung des Aerosols austritt und in den zu desinfizierenden oder sanitifizierenden Raum eintritt. Der im Kanal gemessene Wert kann als Schätzwert für die Konzentration des Desinfektionsmittels in der Raumluft verwendet werden. Mittels eines Modells kann zum Beispiel aus dem im Kanal gemessenen Wert ein Wert für die Konzentration in der Raumluft berechnet werden.

Das Mittel zur Erzeugung eines Aerosols kann ein oder mehrere Ultraschallerzeuger aufweisen, mit dem oder denen das flüssige Desinfektions- oder Sanitationsmittel vernebelt werden kann.

Für das erfindungsgemäße Verfahren können mehrere Mittel zur Erzeugung eines Aerosols eingesetzt werden oder es wird nur ein Mittel eingesetzt. Der Vorteil des Einsatzes von mehreren Mittel ist, dass damit, sofern die Mittel an verschiedenen Stellen des Raumes, möglichst gleichmäßig verteilt angeordnet sind, besser eine gleichmäßige Verteilung des Aerosols in dem Raum und damit in dem Raum eine gleichmäßige Aerosolkonzentration erreicht werden kann. Durch den Einsatz von mehreren Mittel kann es aber dazu kommen, dass die Regelung der Konzentration des Desinfektionsmittels in der Raumluft komplexer ist als bei nur einem Mittel. Mit einem dazu programmierten Regelungsmittel kann aber auch eine solche komplexe Regelung geleistet werden.

Sind für das Verfahren mehrere Sensoren vorgesehen, kann das zumindest zwei Vorteile haben. Zum einen kann durch mehrere Sensoren besser überwacht werden, ob es an einer Stelle im Raum zu einer unzulässig hohen Konzentration des Desinfektionsmittels in der Raumluft kommt, um dann mittels des Regelungsmittels das oder die Mittel zur Aerosolerzeugung so einzustellen, dass die Führungsgröße nicht länger überschritten wird. Zum anderen kann mittels mehrerer Sensoren festgestellt werden, wenn die Konzentration des Desinfektionsmittels in der Raumluft an einer Stelle im Raum nach unten von der Führungsgröße abweicht und es so zu einer unnötig verzögerten Desinfektion oder Sanitation der Stelle im Raum kommt. Auch dieses kann dann mittels des Regelungsmittels korrigiert werden, das dazu das oder die Mittel zur Aerosolerzeugung einstellt.

Vorzugsweise bleibt bei der Durchführung des erfindungsgemäßen Verfahrens der Raum während des Austragens des das Desinfektions-oder Sanitationsmittel enthaltenden Aerosols für Menschen betretbar, das kann bedeuten, dass Zugänge zu dem Raum nicht versperrt und/oder abgeschlossen werden, weder durch Personen noch automatisch.

Bei der Nutzung von mehreren Sensoren zur Messung der Konzentration des Desinfektionsmittels in der Luft zu einem Zeitpunkt kann je nach Ausgestaltung des Verfahrens derjenige Messwert für die Konzentration als Regelgröße verwendet werden, der am größten ist. Das kann insbesondere dann der Fall sein, wenn zwar mehrere Sensoren, aber nur ein Mittel zur Aerosolerzeugung vorgesehen ist.

Vorzugsweise wird die Konzentration in einer Höhe von bis zu 2,5 m über von Menschen betretbaren Flächen gemessen. Konzentrationen in nicht vom Menschen erreichbaren Bereichen können höher oder geringer sein. Der Einfluss solcher Abweichungen der Konzentrationen auf das Infektionsrisiko oder die Gesundheit der Nutzer des Raums ist im Vergleich zu solchen Abweichungen in von den Nutzern erreichbaren Bereichen des Raumes gering.

Die Konzentration kann nach einem erfindungsgemäßen Verfahren an verschiedenen, möglichst gleichmäßig in dem Raum verteilten Stellen gemessen werden. So kann verhindert werden, dass an einer Stelle des Raums, die weit von dem womöglich einzigen Sensor ist, eine zu hohe oder niedrige Konzentration entsteht.

Von Vorteil ist es ebenfalls, wenn bei einem erfindungsgemäßen Verfahren das von dem Mittel oder den Mitteln zur Erzeugung des Aerosols erzeugte Aerosol an verschiedenen, möglichst gleichmäßig in dem Raum verteilten Stellen ausgetragen wird. Dazu können die Mittel gleichmäßig in dem Raum verteilt angeordnet sein. Ist nur ein Mittel zur Erzeugung des Aerosols vorgesehen, kann das mehrere Auslässe aufweisen, über das Aerosol ausgetragen werden kann. Diese Auslässe können in dem Raum verteilt angeordnet sein. Das Mittel kann so eingerichtet sein, dass über die Auslässe unterschiedlich große Mengen des Aerosols ausgetragen werden. Dazu kann zum Beispiel zumindest einer der Auslässe ganz oder teilweise verschlossen werden, zum Beispiel mittels einer Klappe, eines Schiebers oder dergleichen.

Bei einem erfindungsgemäßen Verfahren kann das von dem Mittel oder den Mitteln zur Erzeugung des Aerosols erzeugte Aerosol einer Lüftungs- oder Klimaanlage zugeleitet werden, über die es dann in den Raum oder sogar in mehrere Räume ausgetragen wird.

Für das erfindungsgemäße Verfahren kann eine erfindungsgemäße Vorrichtung zur Desinfektion oder Sanitation von Räumen mittels eines Aerosols, dass ein Desinfektionsmittel enthält, genutzt werden. Diese Vorrichtung ist
- mit wenigstens einem Sensor zum Messen einer Konzentration des Desinfektionsmittels in der Raumluft,
- mit einem Behälter für das Desinfektionsmittel, in dem das Desinfektionsmittel in flüssiger Form vorgehalten werden kann,
- mit wenigstens einem Mittel zur Erzeugung des Aerosols aus dem in dem Behälter vorgehaltenen Desinfektionsmittel und aus dem Raum dem Mittel zugeführter Luft und
- mit einem Regelungsmittel zum Regeln der Konzentration des Desinfektionsmittels in der Raumluft, das mit dem Sensor und dem Mittel zur Erzeugung des Aerosols verbunden ist, ausgestattet.
Dabei ist das Regelungsmittel so programmiert, dass es aus der gemessenen Konzentration des Desinfektionsmittels in der Raumluft und einer Führungsgröße eine Stellgröße zum Einstellen des Mittels zur Erzeugung des Aerosols ermittelt, und dass die Führungsgröße einen Arbeitsplatzgrenzwert für die Aerosolkonzentration für das Desinfektionsmittel nicht überschreitet.

Weitere Merkmale und Vorteile der vorliegenden Erfindung werden deutlich anhand der nachfolgenden Beschreibung unter Bezugnahme auf die beiliegenden Abbildungen. Darin zeigen:
- Fig. 1: eine Darstellung eines Regelkreises einer erfindungsgemäßen Vorrichtung zur Durchführung des erfindungsgemäßen Verfahrens.

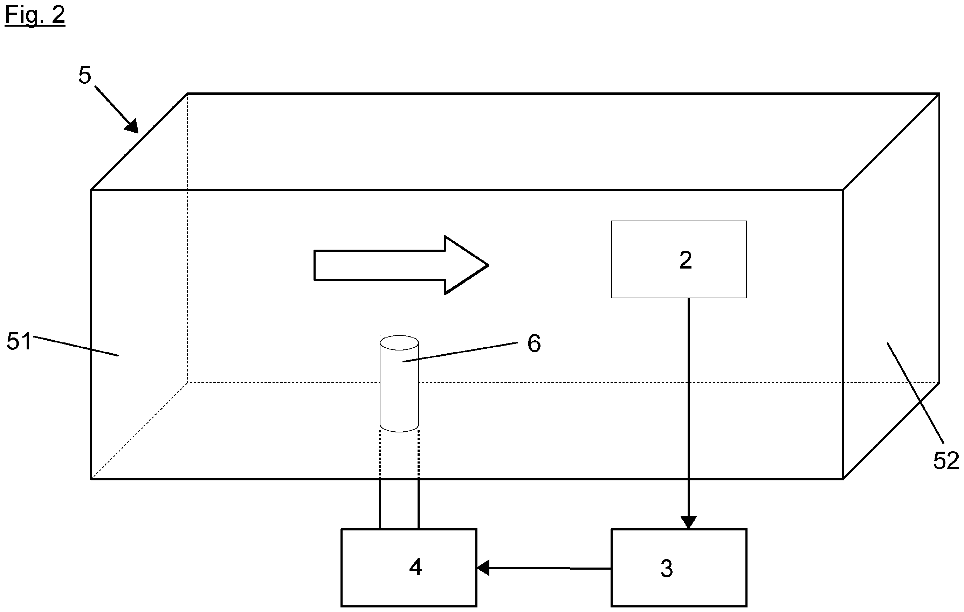

- Fig. 2: eine erste schematische Darstellung einer erfindungsgemäßen Vorrichtung und

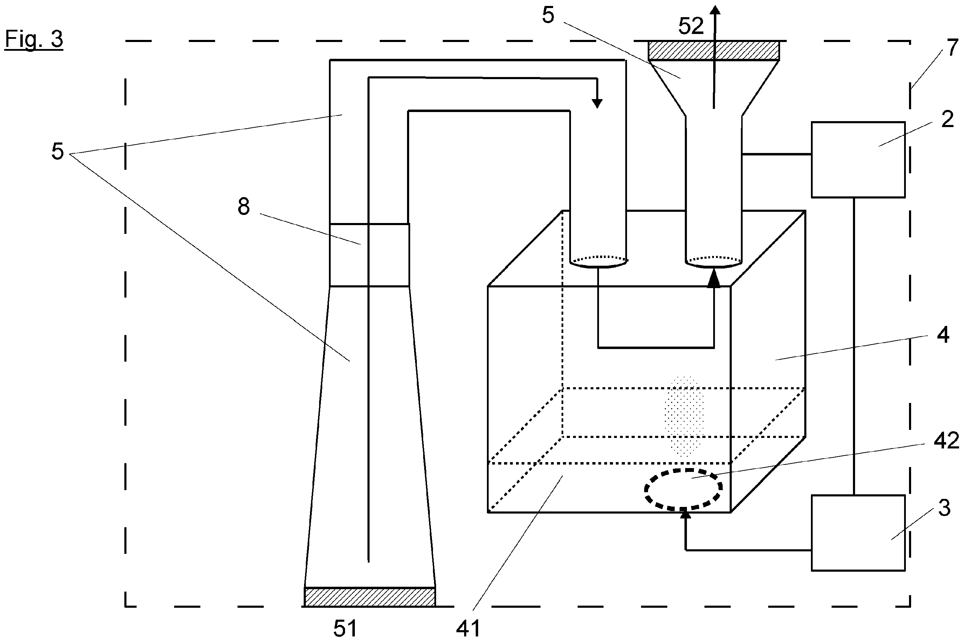

- Fig. 3: eine zweite schematische Darstellung einer erfindungsgemäßen Vorrichtung.

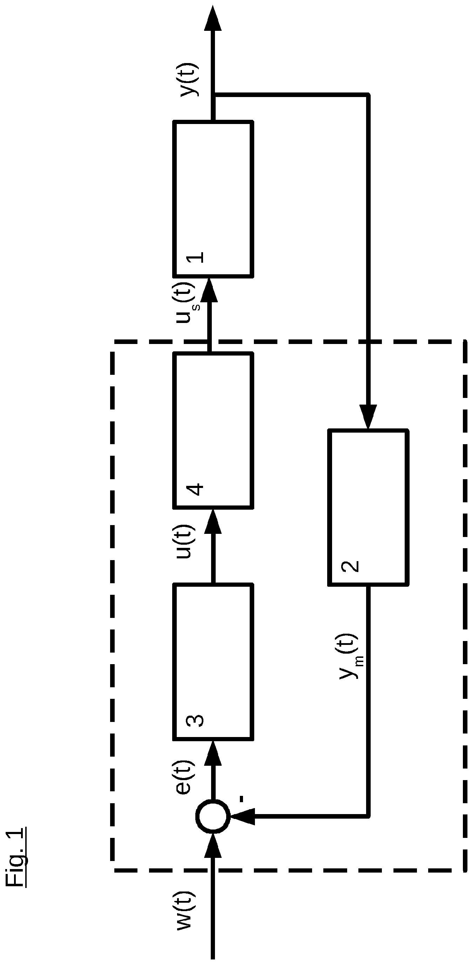

Anhand des in der Fig. 1 dargestellten Regelkreises kann das erfindungsgemäße Verfahren veranschaulicht werden. Durch das Verfahren wird die Konzentration y(t) eines Desinfektionsmittels in der Luft eines Raumes 1 geregelt. Die Konzentration y(t) wird mit einem Sensor 2 erfasst, der ein die Konzentration anzeigendes elektrisches Signal yₘ(t) erzeugt. Anschließend wird durch Differenzbildung eine Regelabweichung e(t) ermittelt, wozu eine Differenz zwischen dem Signal yₘ(t) und einer eine Führungsgröße anzeigenden Signal w(t) gebildet wird. Die Regelabweichung e(t) wird einem Regler 3 zugeführt, der eine Stellgröße u(t) ermittelt. Der Regler kann ein Mikrocontroller sein. Die Stellgröße u(t) wird einem Stellglied zugeführt. Das Stellglied ist ein Mittel 4 zur Erzeugung eines Aerosols aus dem Desinfektionsmittel. Die Stellgröße u(t) könnte die Leistung des Aerosolerzeugers sein, die an dem Mittel 4 eingestellt wird. Durch das Mittel 4 zur Erzeugung des Aerosols wird eine Menge uₛ(t) des Aerosols aus dem Desinfektionsmittel erzeugt und in den Raum 1 abgegeben. In dem Raum vermischt sich das Aerosol mit der Luft. Tröpfchen des Aerosols schlagen sich auch auf Oberflächen in dem Raum nieder. Durch Türen oder Fenster oder Lüftungsöffnungen kann Aerosol aus dem Raum entweichen. Durch das Niederschlagen und das Entweichen kann sich die Konzentration y(t) des Desinfektionsmittels verringern. Durch das Zuführen von Aerosol aus dem Mittel 4 kann die Konzentration erhöht werden.

Gemäß der Erfindung ist vorgesehen, die Konzentration des Desinfektionsmittels so zu Regel, dass er geringer ist als ein Grenzwert für die Konzentration in von Menschen benutzten Räumen. Dazu wird eine Führungsgröße w(t) gewählt, die nicht höher als der Grenzwert ist, vorzugsweise sogar um 10 % geringer als der Grenzwert.

Die erste Darstellung einer erfindungsgemäßen Vorrichtung zeigt im Wesentlichen einen Luftkanal 5, über den Luft aus einem Raum 1 in die Vorrichtung hinein, durch die Vorrichtung hindurch und zurück in den Raum 1 geleitet wird. Zwischen einem Einlass, über den die Luft aus dem Raum 1 in den Luftkanal 5 eintritt und einem Auslass 52, durch den die Luft aus dem Luftkanal 5 in den Raum 1 austritt, mündet ein Kanal 6 in den Luftkanal 5, der den Luftkanal 5 mit einem Mittel 4 zur Erzeugung des Aerosols verbindet. In dem Luftkanal 5 ist ein Sensor 2 zum mittelbaren Messen der Konzentration des Desinfektionsmittels in Raumluft vorgesehen. Dieser Sensor 2 und das Mittel 4 zur Erzeugung sind mit einem Regelungsmittel 3 verbunden. Das Regelungsmittel 3 empfängt von dem Sensor 2 einen Messwert, der die Konzentration des Desinfektionsmittels anzeigt. Das Regelungsmittel 3 ermittelt aus dem Messwert und einer vorgegebenen Führungsgröße eine Stellgröße. Diese Stellgröße wird dem Mittel 4 zur Erzeugung zugeführt, das die notwendige Menge des Desinfektionsmittels zerstäubt, die notwendig ist, um die gewünschte Konzentration zu erreichen.

Der Luftkanal 5, das Mittel 4 zur Erzeugung, der Sensor 2 und das Regelungsmittel 3 können in einem Gehäuse 7 der erfindungsgemäßen Vorrichtung angeordnet sein, das die zweite Darstellung zeigt. In dem Gehäuse 7 ist ferner ein Lüfter 8 angeordnet, der dafür sorgt, dass die Luft aus dem Raum am Einlass 51 angesaugt und durch den Luftkanal 5 an dem Mittel zur Erzeugung 4 vorbei zum Auslass 52 gefördert wird. Außerdem ist dargestellt, dass das Mittel 4 zur Erzeugung eine Wanne 41 aufweist, in der das Desinfektionsmittel vorgesehen ist. An dem Boden der Wanne 41 ist ein Ultraschallzerstäuber 42 angeordnet, mit dem das Desinfektionsmittel zerstäubt und in ein Aerosol verwandelt werden kann. Der Ultraschallzerstäuber 42 ist mit dem Regelungsmittel 3 verbunden und wird von diesem angesteuert.

## Patentansprüche

1. Verfahren zur Sanitation oder Desinfektion in einem Raum (1) mit wenigstens einem Aerosol, das Tröpfchen eines flüssigen Desinfektionsmittels enthält, wobei
- eine Konzentration (y(t)) des Desinfektionsmittels in der Luft des Raumes (1) unmittelbar oder mittelbar geregelt wird,
- eine zu erreichende Konzentration des Desinfektionsmittels in der Luft des Raumes (1) als Führungsgröße (w(t)) festgelegt wird,
- eine Konzentration (y(t)) des Desinfektionsmittels in der Luft des Raumes (1) mit wenigstens einem Sensor (2) unmittelbar oder mittelbar kontinuierlich oder zu diskreten Zeitpunkten erfasst wird und eine Regelgröße bildet,
- aus der erfassten Konzentration (y(t)) und der zu erreichenden Konzentration (w(t)) mit einem Regelungsmittel (3) kontinuierlich oder zu diskreten Zeitpunkten wenigstens eine kontinuierliche oder diskrete Stellgröße (u(t)) ermittelt wird,
- an wenigstens einem Mittel (4) zur Erzeugung des Aerosols fortlaufend die kontinuierliche oder diskrete Stellgröße (u(t)) eingestellt wird, das wenigstens eine Mittel (4) zur Erzeugung des Aerosols dann entsprechend der Einstellung aus einer einem Vorrat des Desinfektions- oder Sanitationsmittels entnommenen Menge des Desinfektions-oder Sanitationsmittels und aus dem Raum zugeführter Luft das Aerosol erzeugt und das erzeugte Aerosol in den Raum (1) austrägt,
**dadurch gekennzeichnet, dass**
- die zu erreichende Konzentration (w(t)) des Desinfektionsmittels in der Luft des Raumes (1) geringer oder genauso groß ist wie ein Grenzwert für die Aerosolkonzentration für das Desinfektionsmittel.

2. Verfahren nach Anspruch 1, **dadurch gekennzeichnet, dass** der Raum (1) während des Austragens des das Desinfektions- oder Sanitationsmittel enthaltenden Aerosols für Menschen betretbar bleibt.

3. Verfahren nach Anspruch 1 oder 2, **dadurch gekennzeichnet, dass** bei der Nutzung von mehreren Sensoren (2) zur Messung der Konzentration des Desinfektionsmittels in der Luft zu einem Zeitpunkt derjenige Messwert für die Konzentration als Regelgröße verwendet wird, der am größten ist.

4. Verfahren nach einem der Ansprüche 1 bis 3, **dadurch gekennzeichnet, dass** die Konzentration in einer Höhe von bis zu 2,5 m über von Menschen betretbaren Flächen gemessen wird.

5. Verfahren nach einem der Ansprüche 1 bis 5, **dadurch gekennzeichnet, dass** die Konzentration an verschiedenen, möglichst gleichmäßig in dem Raum (1) verteilten Stellen gemessen wird.

6. Verfahren nach einem der Ansprüche 1 bis 6, **dadurch gekennzeichnet, dass** das von dem Mittel (4) oder den Mitteln zur Erzeugung des Aerosols erzeugte Aerosol an verschiedenen, möglichst gleichmäßig in dem Raum verteilten Stellen ausgetragen wird.

7. Verfahren nach einem der Ansprüche 1 bis 7, **dadurch gekennzeichnet, dass** das von dem Mittel (4) oder den Mitteln zur Erzeugung des Aerosols erzeugte Aerosol einer Lüftungs-oder Klimaanlage zugeleitet wird, über die es dann in den Raum ausgetragen wird.

8. Vorrichtung zur Desinfektion oder Sanitation von Räumen (1) mittels eines Aerosols, dass ein Desinfektionsmittel enthält,
- mit wenigstens einem Sensor (2) zum unmittelbaren oder mittelbaren Messen einer Konzentration (y(t)) des Desinfektionsmittels in der Raumluft,
- mit einem Behälter für das Desinfektionsmittel, in dem das Desinfektionsmittel in flüssiger Form vorgehalten werden kann,
- mit wenigstens einem Mittel (4) zur Erzeugung des Aerosols aus dem in dem Behälter vorgehaltenen Desinfektionsmittel und aus dem Raum (1) dem Mittel zugeführter Luft,
- mit einem Regelungsmittel (3) zum Regeln der Konzentration des Desinfektionsmittels in der Raumluft, das mit dem Sensor (2) und dem Mittel (4) zur Erzeugung des Aerosols verbunden ist,
- wobei das Regelungsmittel (3) so programmiert ist, dass es aus dem der gemessenen Konzentration (y(t)) des Desinfektionsmittels in der Raumluft und einer Führungsgröße (w(t)) eine Stellgröße (u(t)) zum Einstellen des Mittels (4) zur Erzeugung des Aerosols ermittelt,
**dadurch gekennzeichnet, dass**
- das Regelungsmittel (3) so programmiert ist, dass die Führungsgröße (w(t)) einen Grenzwert für die Aerosolkonzentration für das Desinfektionsmittel nicht überschreitet.
